**Europäisches Patentamt**

(19) **European Patent Office**

**Office européen des brevets**

(11) Publication number : **0 249 374 B1**

# (12) EUROPEAN PATENT SPECIFICATION

(45) Date of publication of patent specification :
**18.03.92 Bulletin 92/12**

(51) Int. Cl.⁵ : **A61F 9/02,** G02C 11/08

(21) Application number : **87304842.5**

(22) Date of filing : **02.06.87**

(54) **Goggle Wiper.**

(30) Priority : **06.06.86 US 871448**

(43) Date of publication of application :
**16.12.87 Bulletin 87/51**

(45) Publication of the grant of the patent :
**18.03.92 Bulletin 92/12**

(84) Designated Contracting States :
**AT CH DE FR LI**

(56) References cited :
**DE-A- 2 506 183**
**US-A- 2 380 855**
**US-A- 3 178 747**
**US-A- 3 929 345**
**US-A- 4 342 128**

(73) Proprietor : **Siler Jr., Lawrence L. "Buzz"**
**3328 Lakeview Boulevard**
**Lake Oswego Oregon 97034 (US)**
Proprietor : **Siler, Richard R. "Rick"**
**Route 4, Box 122**
**Hillsboro Oregon 97123 (US)**
Proprietor : **Siler, Sandra L. (Mrs. Buzz)**
**3328 Lakeview Boulevard**
**Lake Oswego Oregon 97034 (US)**
Proprietor : **Siler, Sally Jo (Mrs. Rick)**
**Route 4, Box 122**
**Hillsboro Oregon 97123 (US)**

(72) Inventor : **Siler, Lawrence L., Jr.**
**3328 Lakeview Boulevard Lake Oswego**
**Clackamas County Oregon 97034 (US)**
Inventor : **Siler, Richard R.**
**Route 4, Box 122 Hillsboro**
**Washington County Oregon 97123 (US)**

(74) Representative : **Parker, Nigel Edward et al**
**H.N. & W.S. Skerrett Rutland House 148,**
**Edmund Street**
**Birmingham B3 2LQ (GB)**

## Description

The present invention, generally, is a wiper to remove snow from goggles as disclosed in the US-A-4342128, and, more specially, is a fixture including a wiper means to be fastened onto a user's thumb, wrist, ski pole, or the like, so that the user can easily remove, from his or her ski goggles or other vision protection devices, snow and other undesireable matter that would tend to obscure vision.

## BACKGROUND OF THE INVENTION

Skiers and other snow sports enthusiasts are constantly vexed by the problem presented by snow, sleet, freezing rain, slush and other objectionable matter collecting on their goggles during use, and by the need to remove this matter promptly, without stopping their activity. This problem is especially irritating when snow is wet and sticky. Not doing something about it immediately upon perception of the problem exposes skiers to the real danger of injury, to themselves and others. Many skiers give up the battle and don't wear goggles, or push them onto their foreheads or hats where they provide no protection. Others use their sleeves, the back of their hands or gloves, kerchiefs, etc., to try and keep their goggles clean during skiing. However, none of these efforts is particularly effective or satisfactory, and few efforts, if any, have been made to solve the specific problem.

Needed is a means of removing snow from ski goggles which can be easily and conveniently carried, which doesn't interefere with a skier's mobility when not needed, and which is available for use immediately upon perception that the goggles need to be cleaned, and without any complex procedure or thought required, if possible.

There is little prior art relating to utensils for the removal of foreign material from glass and other fragile materials, such as the plastics used in ski goggles and glasses, which can also be easily carried until needed, and then used with a minimum of delay and effort.

Of course, the problem of cleaning unwanted matter from various objects and surfaces has long been recognized, and there are at least as many solutions to the problem as there are types of objects to clean. It is instructive to examine some of the proposed solutions to these problems in order to appreciate the solution represented by the present invention.

Luttinger U.S. Design Patent 55,290 discloses a design for a cleaner for carbide lamps.

The following U.S. patents all disclose scragers or wipers for cleaning kitchen utensils and dishes:

Norton U.S. Design Patent 26,798
Hoffman U.S. Design Patent 34,727
Schwartz U.S. Design Patent 163,774
Lower U.S. Patent 2,380,855

Peterson U.S. Design Patent 199,962 and U.S. utility patent 3,178,747 disclose a shaped flexible multi-purpose scraper or wiper.

Other patents discloses various designs for wipers or scrapers to remove ice, mud and/or dirt from various items and places:

Crossman U.S. Design Patent 27,141 - wheels;

Baldwin U.S. Design Patent 193,679 - golf balls;

Young U.S. Design Patent 269,944 - gasoline nozzle.

The uses of some of these scrapers are only distantly related to the use herein dealt with. However, it,is significant that although most of them incorporate holes for inserting fingers to help hold the tool during use, not one of them suggests, or even infers, the possiblity of fastening the scraper or wiper semi-permanently on the hand, wrist or on another tool used in the procedure associated with the cleaning process, during the time that the scraper or wiper might be needed.

Of course, everyone is acquainted with windshield wipers and the squeegee tools used to clean windows. The efforts of inventors to adapt such devices to eyewear has long been a subject for cartoons. Nevertheless little, if anything, appears in the literature about such tools which could be easily carried when not needed, but instantly available for cleaning glasses and goggles when needed.

None of the above even addresses the problems solved by the present invention, which is to have conveniently available, for use whenever it is needed, an easily used means of removing, from ski goggles and the like, snow, slush, sleet, ice, rain, and other matter which would interfere with the skier's vision during skiing.

US-A-4342128 shows a wiper for removing snow or the like from goggles. It has a handle portion 11 and a wiper 21 secured thereto by an adhesive.

## BRIEF DESCRIPTION OF THE PRESENT INVENTION

The present invention is a novel scraper or wiper, to be attached to a mounting member, which may be a thumb, wrist, glove, ski pole or the like of a skier, for the purpose of wiping snow, slush, sleet, ice, rain (frozen or otherwise), and other material from the glasses or goggles of such persons which might interfere with their vision when engaged in skiing, sledding, tobogganing or the like actitivities.

According to the present invention and starting from US-A-4 342 128 there is provided a wiper for removing vision-obstructing matter from the surface of ski-goggles, said wiper:-

a) having a body member, said body member having a first end and a second end and said first end having thereon a single-edged wiper blade to wipe the surface of the ski-goggles and lying in the plane of said body member, the second end of the body member having holder means, the wiper being characterised by:-

1. said holder means being adapted to be removably mounted on a mounting member having a longitudinal axis, said holder means being generally of circular form and adapted for gripping or fastening onto the mounting member,

2. said body member being substantially planar and being:-

    a. a flipper fabricated of a soft flexible material, and,

    b. integrally formed with the holder means,

3. the edge of said blade extending generally across the holder means and in a direction, in use, across the axis of the mounting member and said edge being contoured to be suited to fit said surface of the ski goggles.

Further according to the present invention there is provided the combination of a wiper and a pair of goggles, said wiper being for removing vision obstructing matter from the surface of the goggles, said wiper:-

a) having holder means connected to a body member having a first end and a second end and said first end having thereon a single-edged wiper blade lying in the plane of said body member, said combination being characterised by:-

1. the wiper being adapted to be removably mounted on a mounting member having a longitudinal axis and said holder means being generally of circular form and adapted for removably fitting on said mounting member and gripping thereto,

2. said body member being a substantially planar body member and said wiper blade lying in the plane of the body member, and said body member being:-

    (a) a flipper fabricated of a soft flexible material, and

    (b) integrally formed with the holder means,

3. the edge of said blade being curved and said edge extending in a direction generally across the holder means and, in use, across the axis of the mounting member and spaced from said axis.

Still further according to the present invention there is provided use of wiper means for removing vision obstructing matter from goggles by wiping the goggles with said wiper means, characterised by using the wiper means in the form of a flipper of soft flexible material removably mounted by gripping holder means on a mounting member, for example a thumb, wrist or handle end of a ski-pole, said flipper having a wiping edge extending transversely to the longitudinal axis of said mounting member and said goggles being wiped by said wiping edge of a wiper blade of the flipper which is contoured to match the surface of said goggles.

The wiper means or flipper may be of a pliable soft rubber.

The holder means may be a circular band or a flexible strip with fastener means thereon, for the purpose of fastening the wiper on a mounting member, which can be a thumb or wrist (directly or over clothing, and with or without gloves), or around the top of a ski pole, so that when the user feels the need to wipe the undesired matter away, he simply raises the wiper up and gives the surface of the goggles one or more swipes to accomplish the purpose.

Embodiments of the present invention may have several unexpected advantages:

– it may be easy to use

– it may be always ready for use when needed;

– the user may not have to stop his activities to use it;

– it may not need to be withdrawn from the pocket to use;

– it may not have to be examined to make sure that the blade is oriented properly;

– it may be of a small size which will not interfere with the skier's normal activities;

– it may be of low weight which will not fatigue the user;

– it may be made of flexible rubber, so that it poses little danger if the user falls upon it or inadvertently jabs it toward his eyes;

– it may represent no great loss is mislaid, lost, stolen or destroyed.

These advantages will become apparent from the description which follows, taken in conjunction with the accompanying drawings.

## BRIEF DESCRIPTION OF THE DRAWINGS

FIGURE 1 discloses an isometric view of one embodiment of the present invention, with a holder means thereon for slipping over the thumb or ski-pole of a user.

FIGURE 2 discloses an isometric view of another embodiment of the present invention, with a holder means thereon for fastening the invention on the wrist of a user.

FIGURE 3 discloses another embodiment for fastening on the handle end of a ski pole.

## DETAILED DESCRIPTION OF THE PRESENT INVENTION

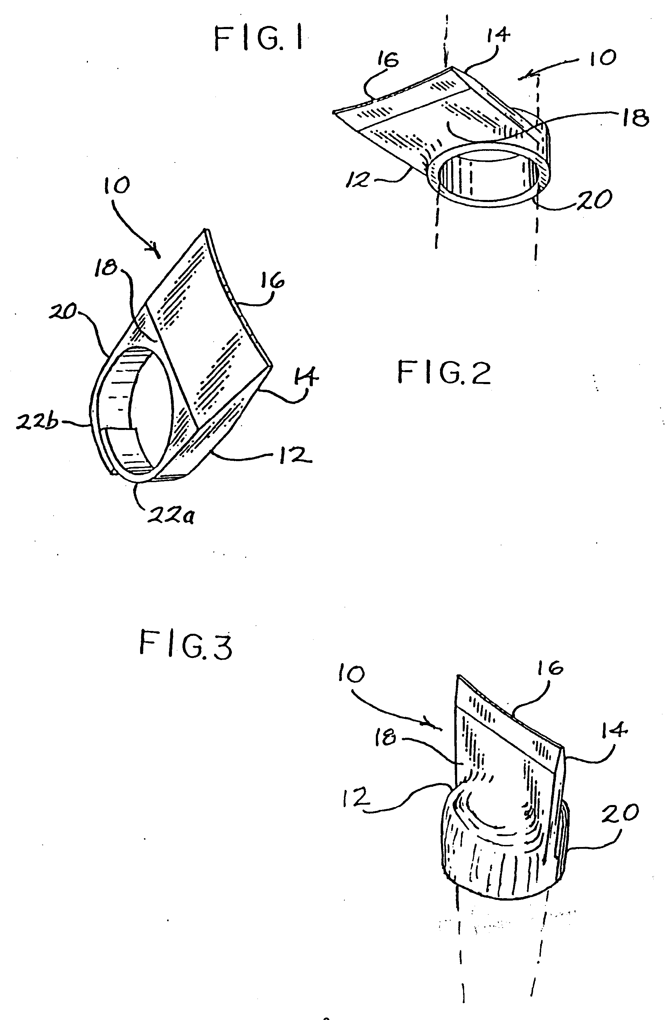

In accordance with the present invention a goggle wiper 10, as disclosed in FIGURE 1, includes a wiper means or flipper 12, composed of a semi-soft, flexible rubber blade 14 having a wiping edge 16 thereon, body 18, and holder means 20. Holder means 20 is intended to be mounted on a mounting member, which can be the thumb of a skier, or the end of a ski-pole.

In use, holder means 20 is slipped over the thumb, for example, after the skier is ready to start skiing, and is adjusted thereon so that, when the hand is raised to the face, the wiping edge 16 of blade 14 contacts the surface of the goggles.

The wiping edge 16 can have a curve therein which is complementary to the curved surface of the ski goggles, as shown.

The size of wiper means 12, and its extension from the skier's hand, are small enough to offer little or no interference with the skier's normal actions. If it is snowing, or if the skier has brushed against a tree laden with snow, so that his goggles become covered with snow and his vision is impaired, all he has to do is brush wiper means 12 across his goggles and his vision is restored. His brushing movement is similar to the normal gesture of wiping one's hand or sleeve across one's eyes so that it can easily become habitual.

The same is true of the embodiment intended to fasten to the skier's wrist, disclosed in FIGURE 2. This embodiment has much the same form and shape as that disclosed in FIGURE 1, except it is slightly larger and the holder means 20 includes a pair of straps 22a and 22b having snaps, Velcro (trade mark) or the like surface contact fastening means thereon for affixing this embodiment snugly to the skier's wrist.

For the ski pole mounting, holder means 20 can have any of several configurations, including cuplike fastener means 24, as disclosed in FIGURE 3, which slips over the end of the ski-pole. It will be apparent that this embodiment might pose a hazard to the skier's eye if the ski pole should encounter an obstacle just at the moment of use.

It will be seen that blade 14 of the wiper means is affixed more-or-less transversely to the axis of the thumb, wrist or ski pole. Especially for the thumb or wrist, this orientation permits the desired wiping motion which is the most natural for the skier.

It will be apparent to those skilled in the art that this invention could take many forms or embodiments without departing thereby from the scope of the claims. For example, gloves, mittens, cuffs or sleeves could have specially adapted mounting fixtures tailored thereto, for holding wiper means 12. Ski poles could be fabricated so that the holder means could be inserted like a cork in the handle end.

It is believed that the relative dimensions of the wipers shown in FIGURES 1 to 3 are particularly advantageous and effective, and advantageous that the wipers may be of simple one-piece construction. It is also advantageous as regards the effectiveness of the wiping action that the wiper is tapered towards its free end in the manner shown in the drawings. It is also possible that the wiper may be formed integrally with the mounting member or effectively permanently attached thereto e. g. by adhesive. Mechanical interlocking means could be provided on the wiper and mounting member. It is also possible that the wiper may be integrated into the design of a glove and be semi-permanently or permanently attached to the glove, or integrally formed therewith.

## Claims

1. A wiper for removing vision-obstructing matter from the surface of ski-goggles, said wiper (10):-a) having a body member (18), said body member having a first end and a second end and said first end having thereon a single-edged wiper blade (14) to wipe the surface of the ski-goggles and lying in the plane of said body member (18), the second end of the body member (18) having holder means (20,22a,22b), the wiper being characterised by:-

    1. said holder means being adapted to be removably mounted on a mounting member having a longitudinal axis, said holder means being generally of circular form and adapted for gripping or fastening onto the mounting member,

    2. said body member (18) being substantially planar and being:-

        a. a flipper fabricated of a soft flexible material, and,

        b. integrally formed with the holder means,

    3. the edge (16) of said blade (14) extending generally across the holder means and in a direction, in use, across the axis of the mounting member and said edge being contoured to be suited to fit said surface of the ski goggles.

2. A wiper (10) as claimed in Claim 1 wherein the holder means (20,22a,22b) is a circumferential band for grippingly sliding over said mounting member.

3. A wiper (10) as claimed in Claim 2 wherein said holder means (20,22a,22b) removably fits onto the thumb of a skier.

4. A wiper (10) as claimed in Claim 2 wherein the holder means (20,22a,22b) fits onto a wrist of the skier.

5. A wiper (10) as claimed in any one of the preceding claims wherein said holder means (20,22a,22b) includes strap ends (22a,22b) with fastening means incorporated thereon.

6. A wiper (10) as claimed in Claim 6 wherein said fastening means is snap fastening means or surface contact fastening means.

7. A wiper (10) as claimed in any one of Claims 1 to 3 wherein said holder means (20) includes cup means for fitting over the end of a ski-pole.

8. A wiper (10) as claimed in any one of Claims 1 to 5 wherein said holder means (20) inserts into the end of a ski-pole.

9. A wiper (10) as claimed in any one of the preceding claims in which the body member (18) is tapered towards the wiping edge (16) thereon, and said body member (18) being of similar width to the width of the holder means (20,22a,22b).

10. The combination of a wiper (10) and a pair of goggles, said wiper (10) being for removing vision obstructing matter from the surface of the goggles, said wiper (10):-a) having holder means (20,22a,22b) connected to a body member (18) having a first end and a second end and said first end having thereon a single-edged wiper blade (14) lying in the plane of said body member (18), said combination being characterised by:

1. the wiper (10) being adapted to be removably mounted on a mounting member having a longitudinal axis and said holder means (20,22a,22b) being generally of circular form and adapted for removably fitting on said mounting member and gripping thereto,

2. said body member (18) being a substantially planar body member (18) and said wiper blade lying in the plane of the body member, and said body member being:-

(a) a flipper fabricated of a soft flexible material, and

(b) integrally formed with the holder means,

3. the edge (16) of said blade (14) being curved and said edge (16) extending in a direction generally across the holder means and, in use, across the axis of the mounting member and spaced from said axis.

11. The combination of a wiper (10), as claimed in Claim 7 or Claim 8 or Claim 9 when dependent therefrom, and a ski pole, the wiper being mounted on the ski pole.

12. Use of wiper means (10) for removing vision obstructing matter from goggles by wiping the goggles with said wiper means (10), characterised by using the wiper means (10) in the form of a flipper (18) of soft flexible material removably mounted by gripping holder means (20,22a,22b) on a mounting member, for example a thumb, wrist or handle end of a ski-pole said flipper (18) having a wiping edge (16) extending transversely to the longitudinal axis of said mounting member and said goggles being wiped by said wiping edge (16) of a wiper blade (14) of the flipper (18) which is contoured to match the surface of said goggles.

**Patentansprüche**

1. Ein Wischer zum Entfernen von die Sicht behinderndern Stoff von der Oberfläche von Skibrillen, wobei der Wischer (10):-a) ein Körperteil (18) aufweist, der einen ersten Endabschnitt und einen zweiten Endabschnitt hat, wobei an dem ersten Endabschnitt ein einseitiges, in der Ebene des Körperteils (18) liegendes Wischerblatt (14) angebracht ist, um die Oberfläche der Skibrille abzuwischen, während an dem zweiten Endabschnitt des Körperteils (18) eine Halterungseinrichtung (20, 22a, 22b) angebracht ist, dadurch gekennzeichnet, daß

1. diese Halterungseinrichtung so gestaltet ist, daß sie lösbar an einern eine Längsachse aufweisenden Halteteil befestigbar ist, im wesentlichen eine Kreisform aufweist und an dem Halteteil festklemmbar oder befestigbar ist;

2. der Körperteil (18) im wesentlichen planar ist und

a) die Form einer aus einem weichen flexiblen Material bestehenden Flosse hat und

b) einstückig mit der Halterungseinrichtung ausgebildet ist, und daß

3. die Kante (16) des Wischerblatts (14) sich im wesentlichen quer zur Halterungseinrichtung und im Gebrauch in einer Richtung quer zur Achse des Halteteils erstreckt, wobei diese Kante eine Kontur hat, die an die Oberfläche der Skibrille angepasst ist.

2. Ein Wischer (10) nach Anspruch 1, bei dem die Halterungseinrichtung (20, 22a, 22b) ein sich in Umfangsrichtung erstreckender Streifen zum festklemmenden Überstreifen über das Halteteil ist.

3. Ein Wischer (10) nach Anspruch 2, bei dem die Halterungseinrichtung (20, 22a, 22b) abnehmbar auf den Daumen eines Skiläufers passt.

4. Ein Wischer (10) nach Anpsruch 2, bei dem die Halterungseinrichtung (20, 22a, 22b) auf das Handgelenk eines Skiläufers passt.

5. Ein Wischer (10) nach einem der vorhergehenden Ansprüche bei dem die Halterungseinrichtung (20, 22a, 22b) Streifenenden (22a, 22b) mit darin eingearbeiteten Befestigungselementen aufweist.

6. Ein Wischer (10) nach Anspruch 5, bei dem diese Befestigungselemente Schnapp-Befestigungselemente oder Oberflächenkontakt-Befestigungselemente sind.

7. Ein Wischer (10) nach einem der Ansprüche 1-3, bei dem die Halterungseinrichtung (20) ein napfförmiges Element umfasst, um auf das obere Ende eines Skistocks zu passen.

8. Ein Wischer (10) nach einem der Ansprüche 1-5, bei dem die Halterungseinrichtung (20) in das Ende eines Skistocks einsetzbar ist.

9. Ein Wischer (10) nach einem der vorhergehenden Ansprüche, bei dem sich der Körperteil (18) zur Wiacherkante (16) hin verjüngt und die gleiche Breite hat wie die Halterungseinrichtung (20, 22a, 22b).

10. Die Kombination eines Wischers (10) und eines Paars von Brillengläsern, wobei der Wischer (10) zum Entfernen von die Sicht behinderndem Stoff von der Oberfläche der Brillengläser dient und der Wischer (10):- a) eine Halterungseinrichtung (20, 22a, 22b) aufweist, die an einem Körperteil (10) mit einem ersten Endabschnitt und einem zweiten Endabschnitt befestigt ist, wobei der erste Endabschnitt ein einseitiges Wischerblatt (14) aufweist, das in der Ebene des Körperteils (18) liegt, wobei diese Kombination dadurch gekennzeichnet ist, daß:

1. der Wischer (10) so gestaltet ist, daß er lösbar an einem einen Längsachse aufweisenden Körperteil befestigt werden kann und die Halterungseinrichtung (20, 22a, 22b) im wesentlichen eine Kreisform hat und lösbar auf das Halteteil passt und daran festklemmbar ist,

2. der Körperteil (18) ein im wesentlichen.planares Körperteil (18) ist, wobei das Wischerblatt in der Ebene des Körperteils liegt, der

a)die Form einer Flosse aus weichem, flexiblem Material hat, und

b)einstückig mit der Halterungseinrichtung ausgebildet ist, und daß

3. die Kante (16) des Wischerblatts (14) kurvenförmig gestaltet ist und sich in einer Richtung im wesentlichen quer zur Halterungseinrichtung und im Gebrauch quer zur Achse des Halteteils erstreckt und im Abstand von dieser Achse liegt.

11.Die Kombination eines Wischers (10) gemäß Anspruch 7 oder Anspruch 8 oder Anspruch 9 und eines Skistockes, wobei der Wischer an dem Skistock befestigt ist.

12.Die Verwendung einer Wischeranordnung (10) zum Entfernen von die Sicht behinderndem Stoff von Brillen durch Abwischen der Brillen mit dieser Wischeranordnung (10), gekennzeichnet durch die Anwendung einer Wischeranordnung (10) in der Form einer Flosse (18) aus weichem, flexiblem Material, die lösbar mittels einer Klemmhalterungeinrichtung (20, 22a, 22b) auf einem Halteteil, beispielsweise einem Daumen, dem Handgelenk oder dem Handgriff eines Skistockes befestigbar ist, wobei diese Flosse (18) eine Wischerkante (16) hat, die sich quer zur Längsachse des Halteteils erstreckt, und wobei diese Brille mittels der Wischerkante (16) eines Wischerblattes (14) der Flosse (18) abwischbar ist, deren Kontur an die Oberfläche der Brille angepasst ist.

## Revendications

1. Raclette pour enlever une matière gênant la vision de la surface de lunettes de ski, ladite raclette (10) comprenant un corps (18), ledit corps comprenant une première et une seconde extrémité et ladite première extrémité possédant une lame d'essuyage à bord unique (14) pour essuyer la surface des lunettes de ski et s'étendant dans le plan dudit corps (18), la seconde extrémité du corps (18) possédant un support (20, 22a, 22b), raclette caractérisée en ce que :

1. ledit support est adapté pour être monté amovible sur une pièce de montage possédant un axe longitudinal, ledit support étant de forme générale circulaire et prévu pour s'accrocher ou se fixer sur la pièce de montage;

2. ledit corps (18) est globalement plat et :

a) fabriqué sous la forme d'une nageoire en un matériau flexible mou et

b) formé intégralement avec le support;

3. le bord (16) de ladite lame (14) s'étendant globalement en travers du support et dans une direction transversale à l'axe de la pièce de montage, en utilisation, ledit bord étant conformé pour s'adapter à ladite surface des lunettes de ski.

2. Raclette (10) selon la revendication 1, dans laquelle le support (20, 22a, 22b) est une bande circonférentielle pour glisser par accrochage sur ladite pièce de montage;

3. Raclette (10) selon la revendication 2, dans laquelle ledit support (20, 22a, 22b) s'adapte de façon amovible sur le pouce d'un skieur.

4. Raclette (10) selon la revendication 2, dans laquelle le support (20, 22a, 22b) s'adapte sur le poignet d'un skieur.

5. Raclette (10) selon l'une quelconque des revendications précédentes, dans laquelle ledit support (20, 22a, 22b) comprend des extrémités de bande (22a, 22b) avec un moyen de fixation incorporé.

6. raclette (10) selon la revendication 6, dans laquelle ledit moyen de fixation est un moyen de fixation par pression ou par contact de surface.

7. Raclette (10) selon l'une quelconque des revendications 1 à 3, dans laquelle ledit support (20) comprend une coupelle s'adaptant sur l'extrémité d'un bâton de ski.

8. Raclette (10) selon l'une quelconque des revendications 1 à 5, dans laquelle ledit support (20) s'insert dans l'extrémité d'un bâton de ski.

9. Raclette (10) selon l'une quelconque des revendications précédentes, dans laquelle le corps (18) est incliné vers le bord d'essuyage (16) et ledit corps (18) est de largeur similaire à celle du support (20, 22a, 22b).

10. Combinaison d'une raclette (10) et d'une paire de lunettes, ladite raclette (10) étant prévue pour enlever toute matière gênant la vision de la surface des lunettes, ladite raclette (10) comprenant un support (20, 22a, 22b) raccordé à un corps (18) possédant une première et une seconde extrémité, ladite première extrémité possédant une lame d'essuyage à bord unique (14) s'étendant dans le plan dudit corps (18), ladite combinaison étant caractérisée en ce que :

1. la raclette (10) est prévue pour être montée amovible sur une pièce de montage possédant un axe longitudinal et ledit support (20, 22a, 22b) est de forme générale circulaire et prévu pour s'adapter de façon amovible sur ladite pièce de montage par accrochage;

2. ledit corps (18) est une pièce globalement plane (18), ladite lame d'essuyage s'étendant dans le plan du corps et ledit corps étant :

    a) fabriqué sous la forme d'une nageoire en un matériau flexible mou et

    b) intégralement formé avec le support;

3. le bord (16) de ladite lame (14) est incurvé, ledit bord (16) s'étendant dans une direction transversale au support et, en utilisation, transversale à l'axe de la pièce de montage de façon espacée.

11. Combinaison d'une raclette (10) selon les revendications 7 ou 8 ou 9, et d'un bâton de ski, la raclette étant montée sur le bâton de ski.

12. Utilisation d'une raclette (10) pour enlever toute matière gênant la vision de lunettes par essuyage avec ladite raclette (10), caractérisée par l'utilisation de la raclette (10) sous la forme d'une nageoire (18) en un matériau flexible mou monté amovible via un support d'accrochage (20, 22a, 22b) sur une pièce de montage, par exemple un pouce, un poignet ou la poignée d'un bâton de ski, ladite nageoire (18) possédant un bord d'essuyage (16) s'étendant transversalement à l'axe longitudinal de ladite pièce de montage et lesdites lunettes étant essuyées par ledit bord d'essuyage (16) d'une lame (14) de la nageoire (18) qui est conformé pour s'adapter à la surface desdites lunettes.

FIG.1

FIG.2

FIG.3